# EUROPEAN PATENT SPECIFICATION

(11) **EP 2 067 433 B1**
(45) Date of publication and mention of the grant of the patent: **01.05.2013**
(21) Application number: 08020975.2
(22) Date of filing: 03.12.2008
(51) Int. Cl.: A61B 1/005

(54) **Medical appliance, endoscope overtube and endoscope apparatus**
Medizinische Vorrichtung, Endoskop und Endoskopvorrichtung
Appareil médical, endoscope et appareil endoscopique

(30) Priority: 03.12.2007 JP 2007312875; 09.01.2008 JP 2008002506
(43) Date of publication of application: 10.06.2009
(73) Proprietor: Olympus Medical Systems Corp., Tokyo 151-0072 (JP)
(72) Inventor: Banju, Kazuo, Tokyo 151-0072 (JP)
(74) Representative: von Hellfeld, Axel

(56) References cited:
- EP-A- 0 165 718
- US-A- 4 762 118
- US-A1- 2007 232 858

## Description

### BACKGROUND OF THE INVENTION

### 1. Field of the Invention

The present invention relates to a medical appliance, an endoscope overtube, and an endoscope apparatus that include a bending mechanism for pulling a bending operation wire inserted through a coil pipe to bend the bending portion of an insertion portion.

### 2. Description of the Related Art

An endoscope having a generally soft insertion portion mainly includes the insertion portion in which a distal end portion, a bending portion, and a flexible tube portion are continuously provided in order from a distal end side, and an operation portion provided on the proximal end side of the insertion portion.

As one example, the bending portion is constituted by linking a plurality of bending pieces. Wire receivers respectively corresponding to an up and down bending operation wire and a left and right bending operation wire are fixedly provided on an inner surface of the bending piece positioned at the most distal end of the bending portion.

The end portions of the bending operation wires are integrally fixed to the respective wire receivers by solder or the like. The middle portions, for example, of the bending operation wires are respectively fixed to pulleys rotatably supported by axes in an operation portion body constituting the operation portion.

Each bending operation wire is inserted through a coil pipe constituted by a closely wound coil or the like. A distal end of the coil pipe is fixed to a coil pipe receiver (not shown) fixed, for example, to the inner surface of the bending piece, and the other end portion is integrally fixed to a coil pipe receiver fixed to the operation portion body. The coil pipe receiver fixed to the operation portion body is integrally fixed with the other end portion of the coil pipe by solder.

In the endoscope having the above-described configuration, when bending knobs provided in the operation portion are operated to rotate the pulleys, the bending operation wires fixed to the pulleys are pulled and loosened, so that the bending portion changes to a desired bending state.

Here, when the pulling and loosening of the bending operation wires inserted through the insertion portion is repeatedly performed for a long period to bend the bending portion, the bending operation wires stretch, or the overall length of the coil pipes shrinks by compressive forces from the bending operation wires, so that the bending operation wires become in a relatively loose state.

Then, the following problems and the like occur: a problem that a change in the bending of the bending portion does not follow the operation of the bending knobs, or a problem that the coil pipes are deformed by the compressive forces from the bending operation wires, so that, for example, the adhesiveness of the proximal end sides of the coil pipes increase, thereby an flexure angle of flexure portions formed in sites fixed to the coil pipe receivers on the other end sides of the coil pipes becomes larger than the original flexure angle of the flexure portions, thereby the amount of a force for pulling the bending operation wires becomes larger.

In order to solve the problem that a change in the bending of the bending portion does not follow the operation of the bending knobs due to the bending operation wires becoming in a relatively loose state, for example, an apparatus for eliminating the loosening of an operation wire in an endoscope that can eliminate the looseness of an operation wire system is shown in Japanese Examined Utility Model Application Publication No. 4-11687.

The apparatus for eliminating the loosening of an operation wire has a configuration in which the looseness of the operation wire system can be eliminated by providing a first adjusting member in a part of a guide sheath, providing in the first adjusting member a second adjusting member for interlocking with the first adjusting member so as to be able to move in a longitudinal direction of the guide sheath, and changing the length of the guide sheath from the variability of the interlocking position in both adjusting members.

On the other hand, in order to solve the problem that the coil pipes are deformed by the compressive forces from the bending operation wires, for example, an approach for using a coil having a diameter larger than a diameter dimension of a coil constituting the coil pipe to constitute a coil pipe is also well known.

Then, by the diameter dimension of the coil being large, the strength of the coil pipe significantly improves, compared with the strength of conventional coil pipes, so that, for example, even when the bending operation wire contacts the coil pipe to produce a compressive force, problems that the proximal end of the coil pipe is crushed, and the like can be solved.

However, in recent years, as a plurality of water feed channels are provided, as a plurality of treatment instrument channels are provided, or as the diameter of the endoscope becomes smaller, the filling factor of internals inserted through the insertion portion increases. Therefore, for expanding space in the insertion portion, it is desired to make a line diameter of the coil constituting the coil pipe small again.

When the coil pipe having a configuration in which the line diameter of the coil is small receives a compressive force from the bending operation wire in the insertion portion through which internals are inserted at a high filling factor, problems that due to the compressive force, for example, the coil pipe intended to be bent is deformed by contacting other internals, and the like are avoided.

However, in the operation portion body, the filling factor of internals is low, so that, for example, when the coil pipes are provided in a generally straight state in the operation portion body, clearance between an outer circumferential surface of the bending operation wire operated to be pulled and an inner circumferential surface of the coil pipe is ensured. Therefore, even when the bending operation wires are repeatedly pulled and loosened, compressive forces is relatively less liable to occur because of a configuration in which the bending operation wires do not easily contact the coil pipes.

On the other hand, for example, when the coil pipes are provided, having a flexure portion in the operation portion body, a contact portion where the outer circumferential surface of the bending operation wire operated to be pulled and the inner circumferential surface of the coil pipe contact to each other appears.

Therefore, when the bending operation wires are repeatedly pulled and loosened, compressive forces are applied to the coil pipes from the bending operation wires, so that the above-described problems may occur.

The present invention has been made in view of the above circumstances and aims to provide a medical appliance, an endoscope overtube, and an endoscope apparatus that prevent coil pipes, through which bending operation wires are inserted, from being deformed by compressive forces from the bending operation wires, so that the pulling of the bending operation wires can be stably performed for a long period.

Documents US 2007/232858 A1, EP 0 165 715 A and US 4,762,118 A disclose medical appliances in which spring coils are used in order to enable movement of a coil pipe along an operation wire extending direction.

### SUMMARY OF THE INVENTION

Briefly, medical appliances according to claims 1 and 2 are provided.

Also, an endoscope overtube according to claim 3 is provided.

Further, an endoscope apparatus according to claim 4 is provided.

The above and other objects, features and advantages of the invention will become more clearly understood from the following description referring to the accompanying drawings.

### BRIEF DESCRIPTION OF THE DRAWINGS

- Fig. 1: is a view explaining a configuration of an overtube externally fitted and arranged outside an insertion portion of an endoscope;
- Fig. 2: is a cross-sectional view along a line II-II in Fig. 1;
- Fig. 3: is a view explaining a configuration of a bending portion of an overtube in Fig. 1;
- Fig. 4: is a view explaining coil pipes through which the bending operation wires of the overtube in Fig. 1 are inserted, and coil pipe supporting members fixedly provided in an operation portion body;
- Fig. 5: is a view explaining a configuration of coil pipe supporting members;
- Fig. 6: is a view explaining an action of the coil pipe supporting members in Fig. 5;
- Fig. 7: is a view explaining a configuration of a treatment instrument that is a medical appliance;
- Fig. 8: is a cross-sectional view along a line VIII-VIII in Fig. 7;
- Fig. 9: is a plan view explaining a configuration of a modification of the coil pipe supporting members;
- Fig. 10: is a side view of Fig. 9 explaining the configuration of the coil pipe supporting members;
- Fig. 11: is a view explaining the modification of the coil pipe supporting members shown in Fig. 9 and Fig. 10;
- Fig. 12: is a plan view explaining a configuration of a further modification of the coil pipe supporting members;
- Fig. 13: is a side view along a line XIII-XIII in Fig. 12, explaining the configuration of the coil pipe supporting members;
- Fig. 14: is a longitudinal cross-sectional view of a further configuration of the coil pipe supporting members; and
- Fig. 15: is a view of a coil pipe receiver in Fig. 14 as seen from a proximal end side.

### DETAILED DESCRIPTION OF THE PREFERRED EMBODIMENTS

The embodiments of the present invention will be described below with reference to the drawings.

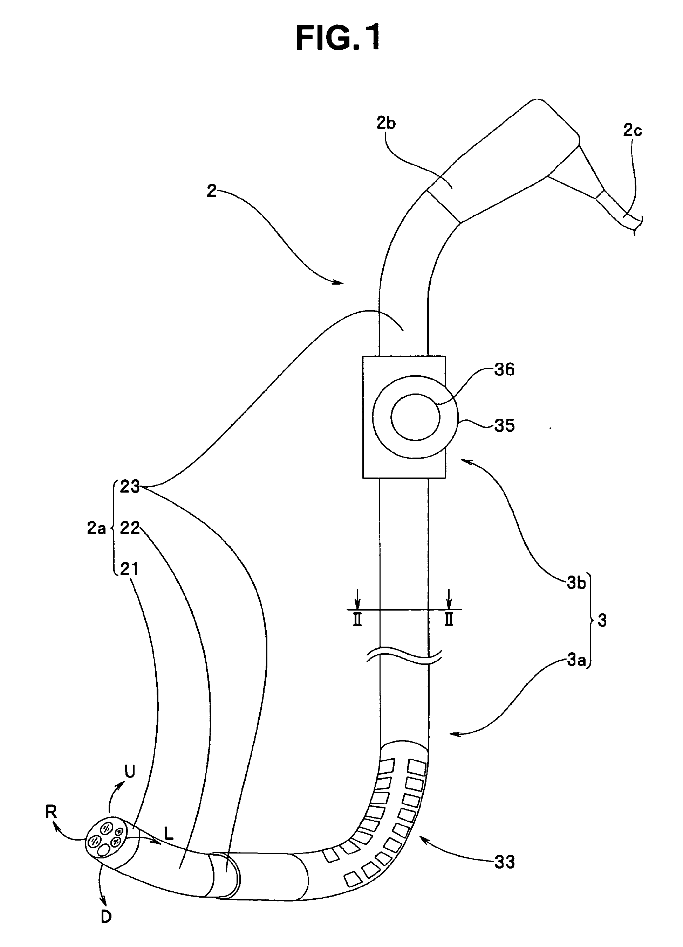

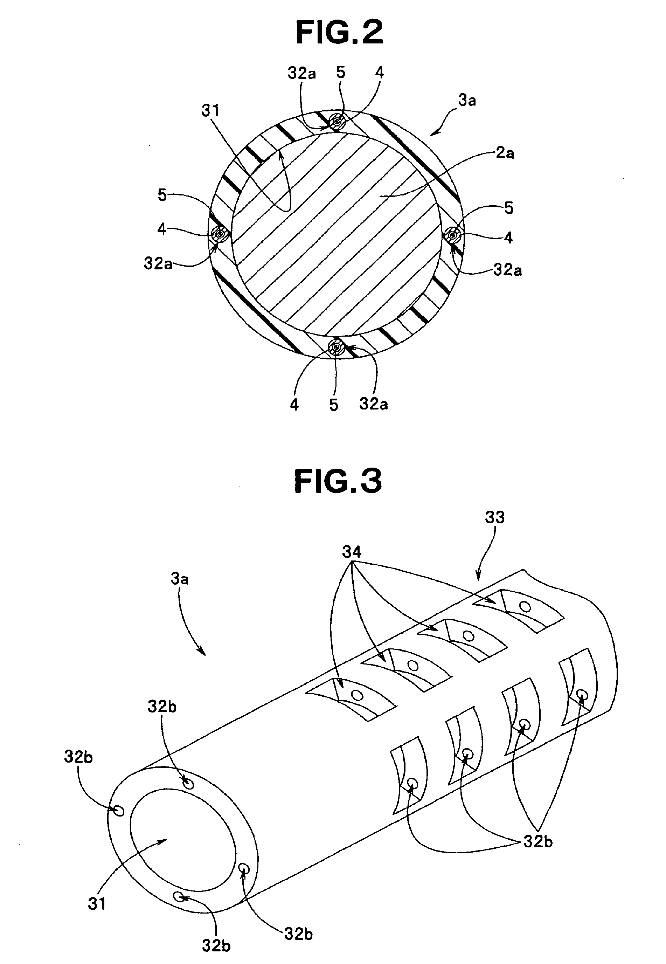

Fig. 1 to Fig. 6 relate to one embodiment of a medical appliance including a bending portion. Fig. 1 is a view explaining a configuration of an overtube externally fitted and arranged outside an insertion portion of an endoscope. Fig. 2 is a cross-sectional view along a line II-II in Fig. 1. Fig. 3 is a view explaining a configuration of the bending portion of the overtube in Fig. 1.

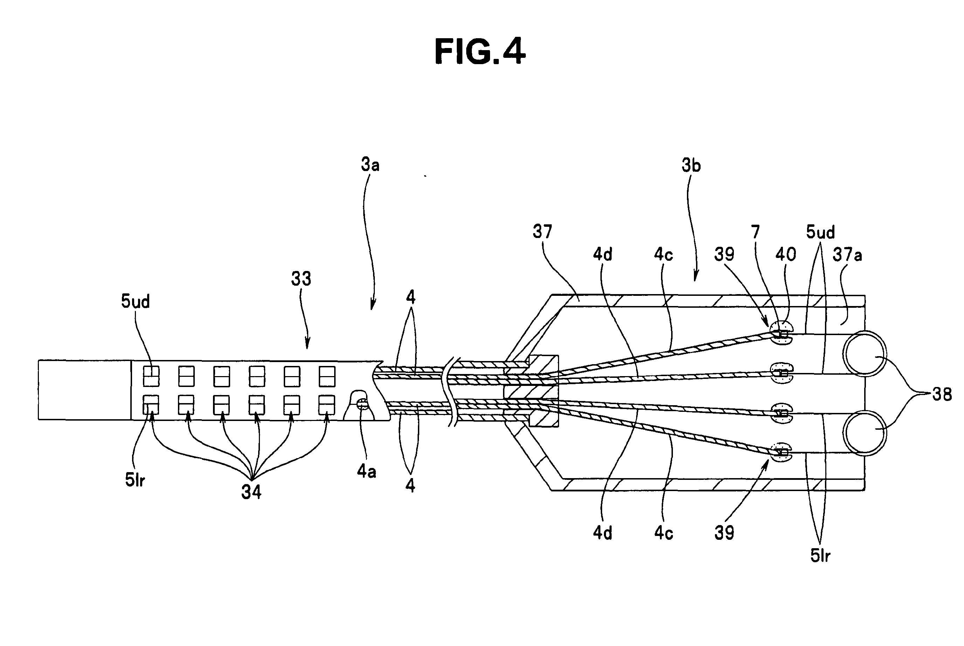

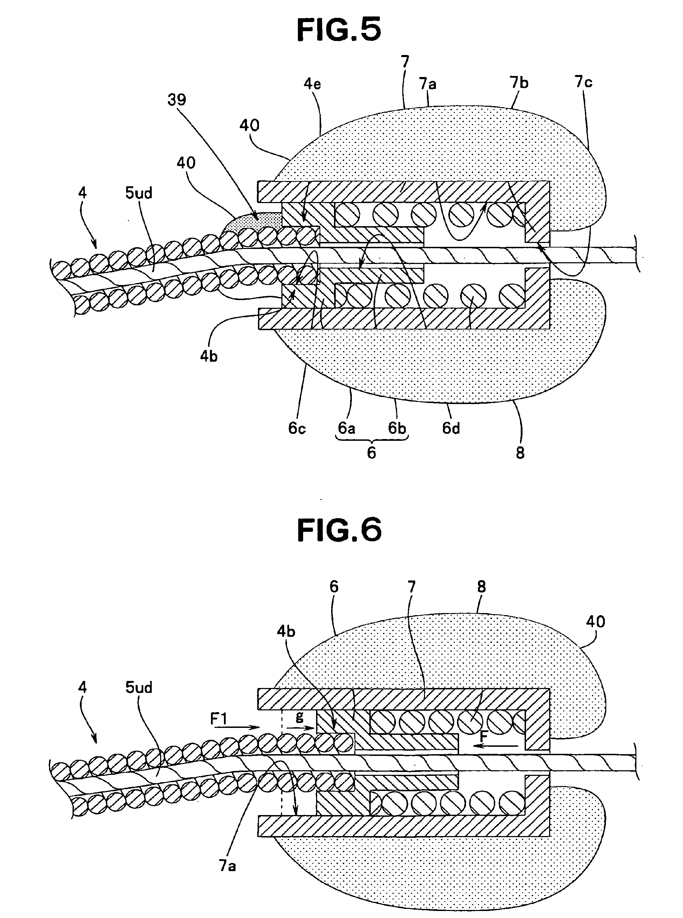

Also, Fig. 4 is a view explaining coil pipes through which the bending operation wires of the overtube in Fig. 1 are inserted, and coil pipe supporting members fixedly provided in an operation portion body. Fig. 5 is a view explaining a configuration of coil pipe supporting members. Fig. 6 is a view explaining an action of the coil pipe supporting members in Fig. 5.

As shown in Fig. 1, an endoscope apparatus 1 of the present embodiment includes an endoscope 2 that is a medical appliance, and an endoscope overtube (hereinafter abbreviated as an overtube) 3 that is a medical appliance.

The endoscope 2 includes an insertion portion 2a, an operation portion 2b, and a universal cord 2c. The insertion portion 2a is constituted by continuously providing a distal end portion 21, a bending portion 22, and a flexible tube portion 23 in order from a distal end side.

The operation portion 2b includes bending knobs (not shown) that are bending operation members. By appropriately operating these bending knobs, bending operation wires (not shown) are operated to be pulled and loosened, so that the bending portion 22 is bent in a desired direction, for example, up and down directions that are a direction of an arrow U and a direction of an arrow D in the figure, or left and right directions that are a direction of an arrow L and a direction of an arrow R.

The overtube 3 is mainly mounted on the flexible tube portion 23 constituting the insertion portion 2a of the endoscope 2. The overtube 3 includes a tube portion 3a and a tube operation portion 3b.

As shown in Fig. 2 and Fig. 3, the tube portion 3a is a multi-lumen tube having flexibility and including a plurality of through holes in a longitudinal axis direction and is composed of silicon, urethane, Teflon (registered trademark), or the like.

The tube portion 3a has an endoscope insertion hole 31 in which the insertion portion 2a of the endoscope is arranged, four, for example, coil pipe insertion holes 32a in which a coil pipe 4 is arranged, and four, for example, bending operation wire insertion holes 32b through which a bending operation wire 5 inserted through a hole in the longitudinal axis direction provided in the coil pipe 4 and protruding from the distal end of the coil pipe 4 is inserted.

An inner diameter dimension of the endoscope insertion hole 31 is larger than an outer diameter dimension of the insertion portion 2a of the endoscope 2 by a predetermined dimension. The four coil pipe insertion holes 32a and bending operation wire insertion holes 32b are provided outside, in an outer circumferential direction, of the endoscope insertion hole 31 and arranged at intervals of, for example, 90 degrees, with respect to a central axis of the endoscope insertion hole 31.

By the bending operation wire 5 being inserted through the coil pipe 4, a portion of the bending operation wire 5 that is inserted through the coil pipe 4 is prevented from directly contacting the tube portion 3a.

As shown in Fig. 1, a tube bending portion 33 having a predetermined length is provided at a predetermined position on a hand side of a distal end of the tube portion 3a. As shown in Fig. 3, the tube bending portion 33 is configured to bend in a desired direction by a predetermined amount by providing a plurality of, rectangular, for example, angular holes 34 for communicating exterior and the endoscope insertion hole 31 at predetermined positions at predetermined intervals. In the present embodiment, the plurality of angular holes 34 are provided in an up direction and a down direction opposed to the up direction in the figure, and a plurality of angular holes 34 are provided in a left direction and a right direction opposed to the left direction in the figure.

As shown in Fig. 1, the tube operation portion 3b is integrally provided at the proximal end of the tube portion 3a. A first knob 35 that is a bending operation member for performing the operation of bending the tube bending portion 33 in the up and down directions, and a second knob 36 that is a bending operation member for performing the operation of bending the tube bending portion 33 in the left and right directions are turnably provided in the tube operation portion 3b.

A distal end portion of the bending operation wire 5 is integrally fixed to the distal end of the tube bending portion 33, in a distal end portion in the hole in the longitudinal axis direction provided in the coil pipe 4, by, for example, adhesion. A distal end portion 4a of the coil pipe 4 in which the distal end portion of the bending operation wire 5 is integrally fixed to the distal end of the tube bending portion 33 is integrally fixed in the coil pipe insertion hole 32a by adhesion or the like, as shown in Fig. 4.

As shown in Fig. 5, a proximal end portion 4e of the coil pipe 4 is integrally fixed to a coil pipe receiver 6 that is a fixing member constituting a coil pipe supporting member, by solder 40 or the like.

The coil pipe receiver 6 to which the proximal end portion 4e of the coil pipe 4 is integrally fixed is provided in a concave portion of a receiving bore 7a of a cylinder 7 that is a cylindrical fixing member receiver that is a coil pipe supporting member.

A compression coil spring 8 that is a coil pipe supporting member and an urging member for urging and supporting the coil pipe receiver 6 is provided in the receiving bore 7a of the cylinder 7 in the present embodiment.

A wire hole 7c through which the bending operation wire 5 is inserted is formed in a side wall 7b corresponding to the bottom portion of the receiving bore 7a constituting the cylinder 7. The wire hole 7c is a through hole for communicating the receiving bore 7a and the exterior of the cylinder 7 and is formed on the longitudinal axis of the cylinder 7.

The coil pipe receiver 6 includes a large diameter portion 6a and a small diameter portion 6b. The large diameter portion 6a is slidably provided in the receiving bore 7a of the cylinder 7. The compression coil spring 8 is installed on an outer circumferential side of the small diameter portion 6b. A pipe bore 6c in which the proximal end portion 4e of the coil pipe 4 is inserted and is provided in the large diameter portion 6a. A wire hole 6d through which the bending operation wire 5 is inserted is formed on a longitudinal axis of the coil pipe receiver 6.

As shown in Fig. 6, an urging force F of the compression coil spring 8 is set considering a compressive force F1 received by the coil pipe 4 from the bending operation wire 5.

As shown in Fig. 4, the cylinder 7 is integrally fixed by the solder 40 at a predetermined position on a partition plate 37a provided in an operation portion body 37 constituting the tube operation portion 3b.

A pair of pulleys 38 are provided in a proximal end portion of the partition plate 37a positioned on the proximal end side of the operation portion body 37. The respective pulleys 38 are configured to turn with the operation of the knobs 35 and 36 provided in the tube operation portion 3b.

A middle portion of an up and down direction bending operation wire 5ud and a middle portion of a left and right direction bending operation wire 5lr are respectively integrally fixed to the respective pulleys 38 by, for example, solder. The bending operation wires 5ud and 5lr are inserted through coil pipes 4c provided to form a flexure portion 39, or coil pipes 4d provided on a generally straight line.

Next, an action of the overtube 3 will be described.

In the overtube 3, for example, by appropriately operating the first knob 35, the up and down direction bending operation wire 5ud is pulled and loosened to bend the tube bending portion 33 in the up and down directions.

Then, the distal end portion 21 of the endoscope 2 protruding from the distal end of the overtube 3 is moved in a direction of an arrow U or a direction of an arrow D in Fig. 1. Then, by moving a first fixing knob (not shown) provided in the first knob 35 to a fixed position, a bending state of the tube bending portion 33 in the up and down directions is maintained.

On the other hand, in the overtube 3, by appropriately operating the second knob 36, the left and right direction bending operation wire 5lr is pulled and loosened to bend the tube bending portion 33 in the left and right directions.

Then, the distal end portion 21 of the endoscope 2 protruding from the distal end of the overtube 3 is moved in a direction of an arrow L or a direction of an arrow R. Then, by moving a second fixing knob (not shown) provided in the second knob 36 to a fixed position, the bending state of the tube bending portion 33 in the left and right directions is maintained.

In other words, by maintaining the tube bending portion 33 of the overtube 3 in a desired bending state, the distal end portion 21 of the endoscope 2 protruding from the distal end of the overtube 3 is maintained in a desired direction in a stable state.

For example, during the operation of the first knob 35, from the up and down direction bending operation wire 5ud contacting a vicinity of the flexure portion 39 of the coil pipe 4, a compressive force acts on the coil pipe 4. Then, as shown in Fig. 6, when the compressive force, shown by an arrow F1, acting on the coil pipe 4 exceeds the urging force F of the compression coil spring 8, the coil pipe receiver 6 in which the proximal end portion 4e of the coil pipe 4 is fixedly provided moves in the receiving bore 7a of the cylinder 7 in the direction of an arrow g, that is, toward the proximal end side on which the pulleys 38 are provided, along a direction in which the bending operation wire extends.

Then, by the coil pipe receiver 6 resisting the urging force F of the compression coil spring 8 to be moved from a position shown by a broken line to a position shown by a solid line, the compressive force acting on the coil pipe 4 from the up and down direction bending operation wire 5ud is released. Therefore, the coil pipe 4 is prevented from being deformed by the compressive force.

Subsequently, when the compressive force F1 acting on the coil pipe 4 from the up and down direction bending operation wire 5ud becomes smaller than the urging force F of the compression coil spring 8, by the urging force of the compression coil spring 8, the coil pipe receiver 6 in which the proximal end portion 4e of the coil pipe 4 is fixedly provided moves in the receiving bore 7a of the cylinder 7 along the direction in which the bending operation wire extends so as to return to an original position.

The same applies to the left and right direction bending operation wire 5lr and the coil pipe 4, and description is omitted.

In this manner, the coil pipe receiver 6 in which the proximal end portion of the bending operation wire 5 is fixedly provided is provided in the receiving bore 7a of the cylinder 7 including the compression coil spring 8. As a result, when a compressive force larger than the urging force of the compression coil spring 8 acts on the coil pipe 4 from the bending operation wire 5, the coil pipe receiver 6 arranged in the receiving bore 7a of the cylinder 7 is moved to the proximal end side, so that the compressive force acting on the coil pipe 4 from the bending operation wire 5 is released, thereby the coil pipe 4 can be prevented from being deformed by the compressive force from the bending operation wire 5. Thus, the pulling of the bending operation wire 5 is stably obtained for a long period.

In the above-described embodiment, the configuration in which the occurrence of a problem caused by the compressive forces applied to the coil pipes 4 by the bending operation wires 5 for bending the tube bending portion 33 of the overtube 3 that is a medical appliance is prevented by providing the above-described coil pipe supporting members is described.

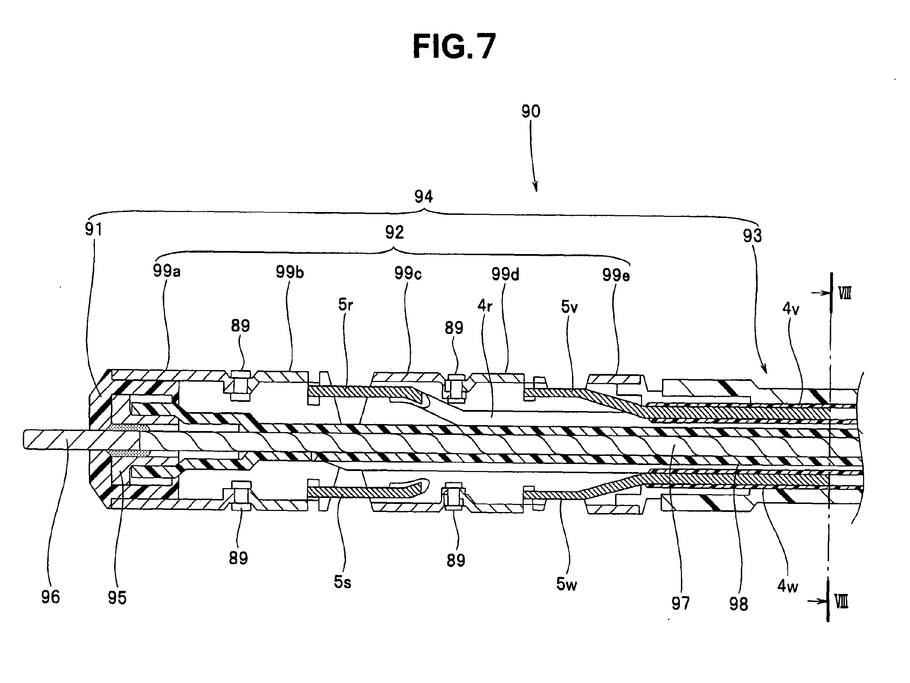

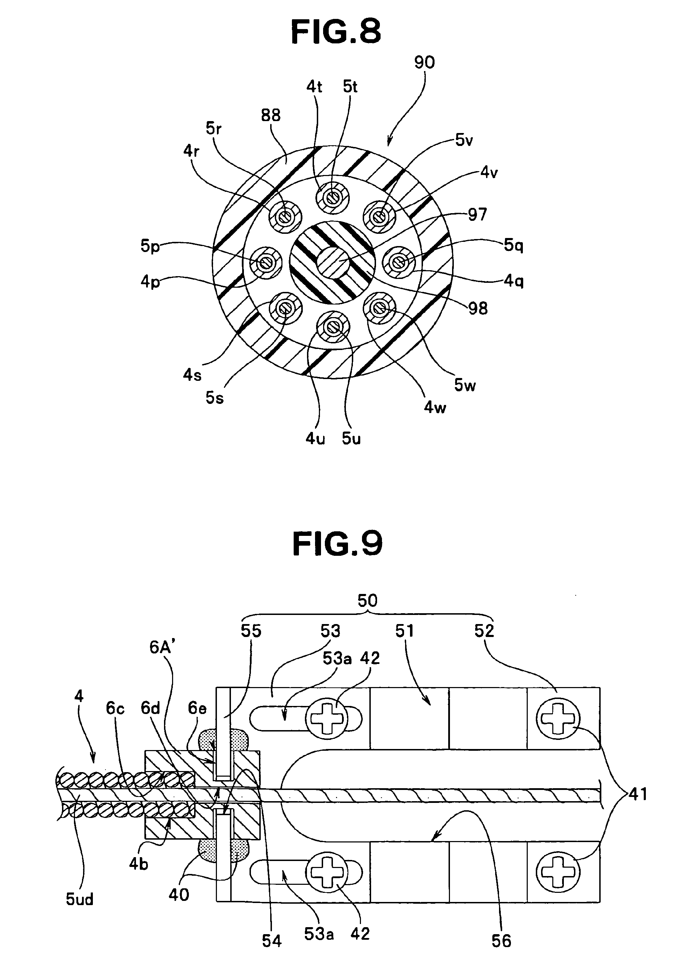

However, the medical appliance in which the above-described coil pipe supporting members are provided is not limited to the overtube and may be an endoscope including bending operation wires for bending a bending portion, and coil pipes through which the wire is inserted, or a treatment instrument inserted through a treatment instrument channel of an endoscope. A configuration of a treatment instrument having a bending portion will be described below with reference to Fig. 7 and Fig. 8.

Fig. 7 and Fig. 8 relate to another embodiment of the medical appliance including a bending portion. Fig. 7 is a view explaining the configuration of a treatment instrument that is a medical appliance, and Fig. 8 is a cross-sectional view along a line VIII-VIII in Fig. 7.

As shown in Fig. 7, a treatment instrument 90 is introduced into a body cavity via the treatment instrument channel of the endoscope 2. The treatment instrument 90 has an insertion portion 94 including a stopper member 91, a bending portion 92, and a flexible tube portion 93. An operation portion (not shown) is connected to a proximal end of the insertion portion 94.

The treatment instrument 90 of the present embodiment is an electric knife. A stopper receiving portion 95 is provided in the stopper member 91, and a knife 96 that is an electrode is integrally fixedly provided in the stopper receiving portion 95.

An electrode operation wire 97 configured to be inserted through a central portion in the insertion portion 94 is connected to a proximal end of the knife 96. A wire cover 98 is provided around the electrode operation wire 97. A distal end portion of the wire cover 98 is fixedly provided in the stopper receiving portion 95.

The bending portion 92 is constituted by continuously providing a plurality of bending pieces 99. The plurality of bending pieces 99 include, for example, a first bending piece 99a, a second bending piece 99b, a third bending piece 99c, a fourth bending piece 99d, and a fifth bending piece 99e in order from a distal end side, and the respective bending pieces are turnably connected by rivets 89 provided in an opposed position relationship.

The distal end portions of bending operation wires 5p, 5q, 5r, 5s, 5t, 5u, 5v, and 5w are respectively fixedly provided at predetermined positions on these bending pieces 99a, 99b, 99c, 99d, and 99e. The proximal end portions of the bending operation wires 5p, 5q, 5r, 5s, 5t, 5u, 5v, and 5w are fixed to pulleys (not shown) provided in the operation portion.

The bending operation wires 5p, 5q, 5r, 5s, 5t, 5u, 5v, and 5w are inserted through coil pipes 4p, 4q, 4r, 4s, 4t, 4u, 4v, and 4w. The proximal end portions (not shown) of the coil pipes 4p, 4q, 4r, 4s, 4t, 4u, 4v, and 4w are fixedly provided in coil pipe receivers 6 provided in the operation portion (not shown) of the treatment instrument 90 and are provided in the receiving bores 7a of cylinders 7 provided in the operation portion (not shown) and including a compression coil spring 8, as in the above-described embodiment.

Thus, a configuration in which the coil pipes 4p, 4q, 4r, 4s, 4t, 4u, 4v, and 4w are prevented from being deformed by compressive forces from the bending operation wires 5p, 5q, 5r, 5s, 5t, 5u, 5v, and 5w, as in the above-described embodiment, is obtained.

Also, in the above-described embodiment, it is shown that the coil pipe supporting members for supporting the coil pipe receiver 6 so as to be able to move in the direction of the pulley 38 are constituted by the cylinder 7, the coil pipe receiver 6 which is provided in the receiving bore 7a of the cylinder 7 and in which the proximal end portion 4e of the coil pipe 4 is fixedly provided, and the compression coil spring 8. However, the coil pipe supporting members are not limited to this configuration and may be configured as shown in Fig. 9 to Fig. 14.

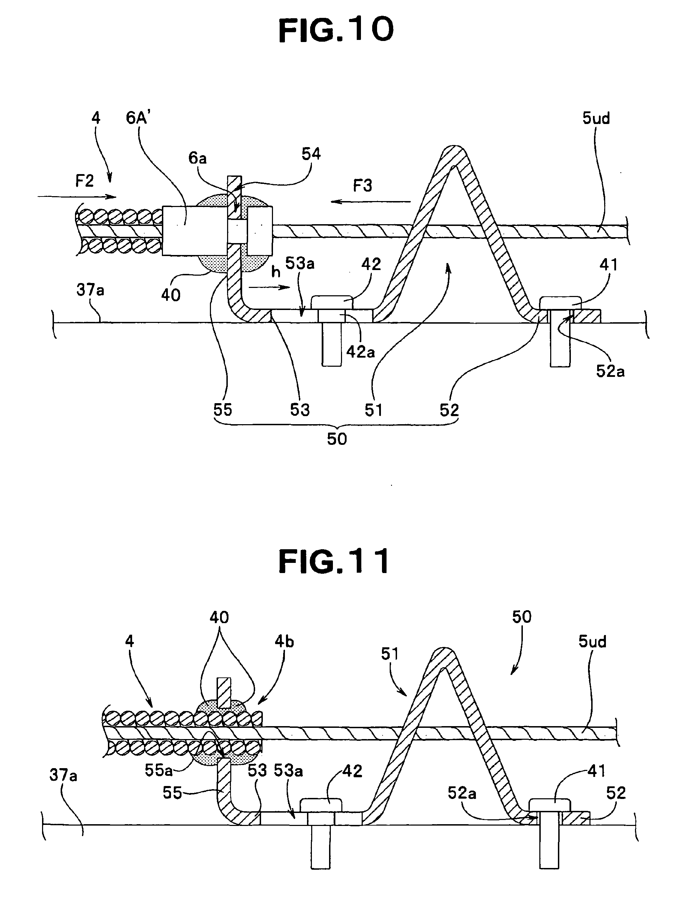

Fig. 9 to Fig. 11 relate to another example of a configuration of coil pipe supporting members. Fig. 9 is a plan view explaining a configuration of a modification of the coil pipe supporting members. Fig. 10 is a side view of Fig. 9, explaining the configuration of the coil pipe supporting members. Fig. 11 is a view explaining the modification of the coil pipe supporting members shown in Fig. 9 and Fig. 10.

As shown in Fig. 9 and Fig. 10, the coil pipe supporting members of the present embodiment are a fixed supporting member 50 in which a coil pipe receiver 6A' is fixedly provided and which includes a plate spring portion 51 that is an urging member.

The coil pipe receiver 6A' is cylindrical and includes on a distal end side a pipe bore 6c in which the proximal end portion 4e of the coil pipe 4 is fixedly provided. A wire hole 6d through which the bending operation wire 5 is inserted is formed on a longitudinal axis of the coil pipe receiver 6A'. A fixing circumferential groove 6e is provided in an outer circumferential surface of the coil pipe receiver 6A'.

The fixed supporting member 50 is constituted by a plate spring portion 51 that is a spring portion, a pair of attachment portion 52 and sliding portion 53, and a supporting portion 55 that is a coil pipe fixing portion including a coil pipe receiver fixing groove (hereinafter abbreviated as a fixing groove) 54. Numeral 56 denotes a notch, and the width of the notch 56 is adjusted to adjust an urging force of the plate spring portion 51.

The plate spring portion 51 is shaped by folding in an inverted V shape between the attachment portion 52 and the sliding portion 53. Holes 52a through which a fixing screw 41 is inserted are formed in the attachment portion 52. The attachment portion 52 is fixed to a tube operation portion 3b by screwing the fixing screw 41.

Long holes 53a in which a step portion 42a of a stepped screw 42 is arranged are formed in the sliding portion 53. The long holes 53a are formed parallel to a longitudinal central axis of the fixed supporting member 50.

The supporting portion 55 is configured to be generally perpendicular to the sliding portion 53. The coil pipe receiver 6A' in which the proximal end portion 4e of the coil pipe 4 is fixedly provided is arranged in the fixing groove 54 formed in the supporting portion 55 and is integrally bonded to the supporting portion 55 by solder 40 or the like.

In the present configuration, a compressive force shown by an arrow F2 acts on the coil pipe 4 from the up and down direction bending operation wire 5ud, and when a compressive force F2 exceeds an urging force F3 of the plate spring portion 51, the supporting portion 55 resists the urging force F3 of the plate spring portion 51 to move to a proximal end side as shown by an arrow h.

Then, the compressive force acting on the coil pipe 4 from the up and down direction bending operation wire 5ud is released, so that the coil pipe 4 is prevented from being deformed by the compressive force. In other words, action and effect similar to those in the above embodiments can be obtained.

Then, when the compressive force F2 acting on the coil pipe 4 from the bending operation wire 5ud becomes smaller than the urging force F3 of the plate spring portion 51, by the urging force F3 of the plate spring portion 51, the coil pipe receiver 6A' in which the proximal end portion 4e of the coil pipe 4 is fixedly provided moves so as to return to an original position.

In the present configuration, the coil pipe 4 is fixed to the coil pipe receiver 6A', and the coil pipe receiver 6A' is bonded to the fixing groove 54 of the supporting portion 55. However, as shown in Fig. 11, the coil pipe 4 may be directly bonded to the supporting portion 55 by the solder 40 or the like.

In this configuration, a through hole 55a through which the coil pipe 4 is inserted is provided in the supporting portion 55, and the coil pipe 4 is bonded by the solder 40, with the proximal end portion 4e of the coil pipe 4 protruding from the through hole 55a by a predetermined amount.

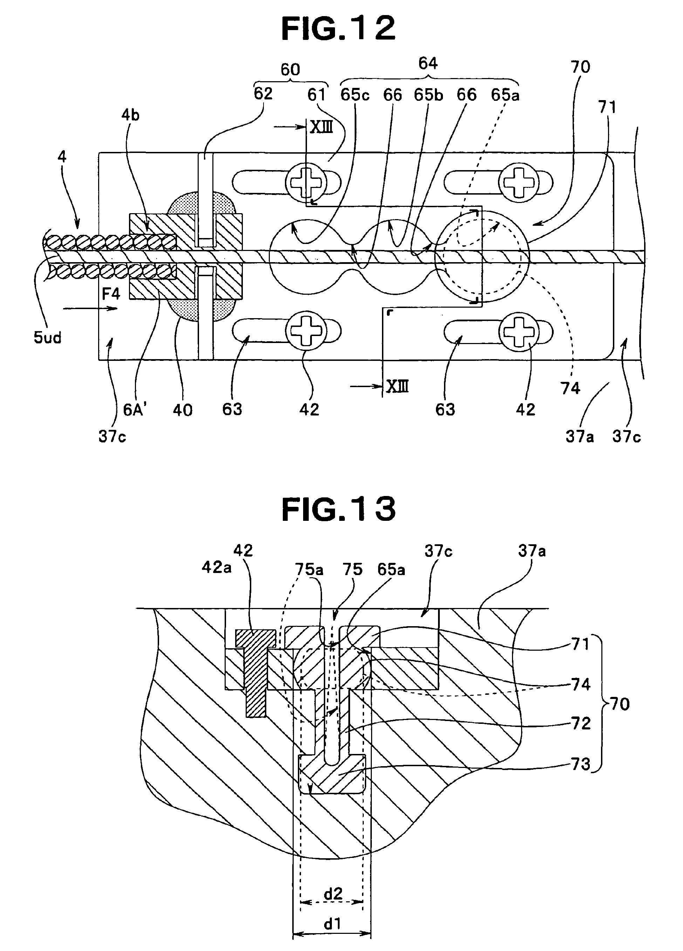

Fig. 12 and Fig. 13 relate to another example of a configuration of coil pipe supporting members. Fig. 12 is a plan view explaining a further configuration of the coil pipe supporting members, and Fig. 13 is a side view along a line XIII-XIII in Fig. 12, explaining the configuration of the coil pipe supporting members.

The coil pipe supporting members in the present configuration are constituted by a sliding supporting member 60 that is a plate-like sliding member in which a coil pipe receiver 6A' is fixedly provided, and a clip 70 that is a fixed supporting shaft for supporting this sliding supporting member 60 so as to be able to move stepwise, shown in Fig. 12 and Fig. 13.

The clip 70 is made of resin and includes a flange portion 71, a shaft portion 72, and a fixed portion 73. A convex portion 74 as an engaging portion protruding with respect to an outer circumference of the shaft portion 72 is provided in the shaft portion 72.

An outer diameter dimension of the convex portion 74 is a first diameter dimension dl. A slit 75 is formed in the flange portion 71 and the shaft portion 72. The fixed portion 73 is press-fitted and arranged in a clip-arranged concave portion 37b provided in a partition plate 37a.

By having the slit 75, the clip 70 is configured so that the flange portion 71 and the shaft portion 72 can be elastically deformed. Specifically, by an external force toward an axis direction acting on the convex portion 74, a slit surface 75a is deformed in an approaching state as shown by a broken line, so that the outer diameter dimension of the convex portion 74 changes to a second diameter dimension d2 smaller than the first diameter dimension.

By releasing the external force toward the axis direction acting on the convex portion 74, the shaft portion 72 and the flange portion 71 of the clip 70 return to an original state. In other words, the diameter dimension of the convex portion 74 changes from the second diameter dimension d2 to the first diameter dimension d1. In the present configuration, the convex portion 74 constitutes an urging member.

The sliding supporting member 60 is configured to be slidable in a sliding support member-installation groove groove 37c formed in the partition plate 37a. The sliding supporting member 60 includes a sliding portion 61 and a supporting portion 62.

Long holes 63 in which a step portion 42a of a stepped screw 42 is arranged are formed in the sliding portion 61. The long holes 63 are formed parallel to a longitudinal central axis of the sliding supporting member 60. A click groove 64 is formed on a longitudinal central axis of the sliding portion 61.

The click groove 64 includes three, for example, bore portions 65a, 65b, and 65c that are engaging insertion portions in which the convex portion 74 having the first diameter dimension d1 is arranged, and a plurality of moving grooves 66 that are moving portions through which the convex portion 74 having the second diameter dimension d2 passes.

The supporting portion 62 is configured to be generally perpendicular to the sliding portion 61. The supporting portion 62 is a coil pipe fixing portion, and a fixing groove (not shown) similar to the fixing groove 54 is formed in the supporting portion 62. The coil pipe receiver 6A' in which the proximal end portion 4e of the coil pipe 4 is fixedly provided is bonded to the fixing groove by solder 40 or the like.

In the present configuration, in an initial state, the sliding supporting member 60 is installed in the sliding support member-installation groove 37c, with the convex portion 74 of the clip 70 press-fitted in the first bore portion 65a of the sliding supporting member 60.

When, for example, a compressive force shown by an arrow F4 acts on the coil pipe 4 from the up and down direction bending operation wire 5ud, in this installed state, and the compressive force F4 increases, a pressing force with which an inner circumferential surface of the first bore portion 65a presses an outer circumferential surface of the convex portion 74, resisting the urging force of the clip 70, increases.

Then, the outer diameter dimension of the convex portion 74 that is the first diameter dimension d1 gradually becomes smaller, and the supporting portion 62 gradually moves to a proximal end side. Thus, the compressive force acting on the coil pipe 4 from the up and down direction bending operation wire 5ud is released, so that the coil pipe 4 is prevented from being deformed by the compressive force.

When successively, the compressive force acts on the coil pipe 4 from the up and down direction bending operation wire 5ud, and the compressive force shown by the arrow F4 further increases, the supporting portion 62 further moves to the proximal end side, and the outer diameter dimension of the convex portion 74 pressed by an inner surface of the first bore portion 65a changes to the second diameter dimension d2, so that the convex portion 74 passes through the moving groove 66 and is press-fitted and arranged in the second bore portion 65b. Thus, the compressive force acting on the coil pipe 4 from the up and down direction bending operation wire 5ud is released, so that the coil pipe 4 is prevented from being deformed by the compressive force.

In other words, in the present configuration, the convex portion 74 of the clip 70 configured to change the diameter dimension is arranged in a predetermined bore portion 65a among the plurality of bore portions 65a, 65b, and 65c of the sliding supporting member 60.

Then, when the compressive force acting on the coil pipe 4 increases, the compressive force acting on the coil pipe 4 from the bending operation wire 5 can be released by the sliding supporting member 60 moving in the sliding support member-installation groove 37c so that the convex portion 74 moves from the bore portion 65a to the bore portion 65b. In other words, action and effect similar to those in the above-described embodiments can be obtained by including the sliding supporting member 60 and the clip 70.

A sensor for detecting that the convex portion 74 of the clip 70 is arranged in the third bore portion 65c may be provided at a predetermined position on the partition plate 37a. Thus, a fact that the sliding supporting member 60 is moved to a final moving point is grasped, so that the replacement of the coil pipe 4, and the like can be performed at appropriate time. Also, in the present configuration, the number of the bore portions 65 formed in the click groove 64 is three, but the number of the bore portions 65 can be appropriately set and may be three or more.

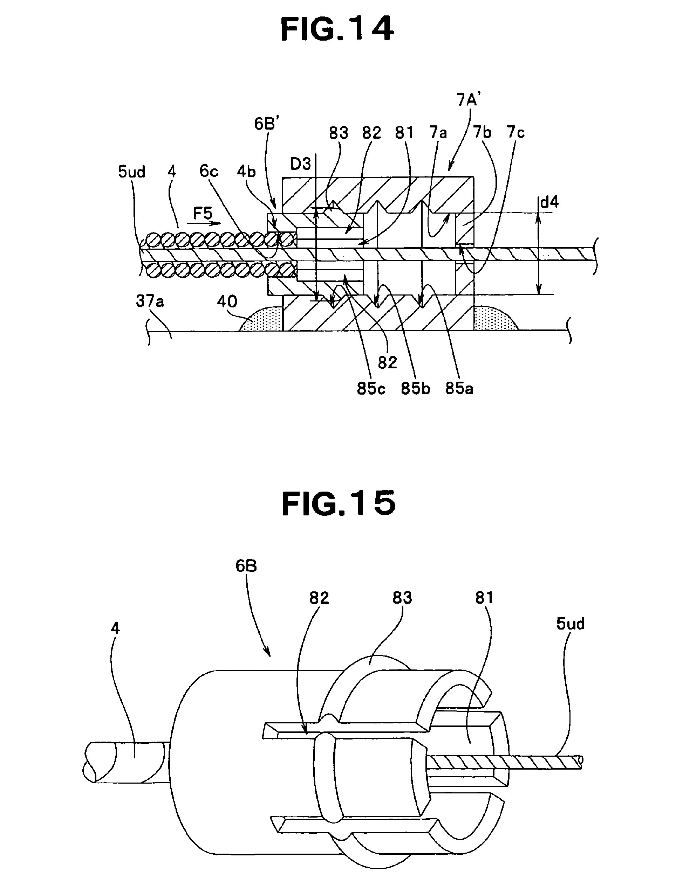

Fig. 14 and Fig. 15 relate to another configuration of coil pipe supporting members. Fig. 14 is a longitudinal cross-sectional view of a further configuration of the coil pipe supporting members, and Fig. 15 is a view of a coil pipe receiver in Fig. 14 as seen from a proximal end side.

The coil pipe supporting members in the present configuration are constituted by a coil pipe receiver 6B' that is a fixing member including an engaging convex portion 83 that is an engaging portion, and a cylinder 7A' that is a fixing member receiver having grooves for supporting the coil pipe receiver 6B' stepwise, as shown in Fig. 14 and Fig. 15.

The coil pipe receiver 6B' is cylindrical, and a pipe bore 6c in which the proximal end portion 4e of the coil pipe 4 is fixedly provided is provided in a distal end portion. A bore portion 81 for elastically deforming a proximal end portion side of the coil pipe receiver 6B', and a plurality of slits 82 for communicating the bore portion 81 and exterior are formed on the proximal end portion side of the coil pipe receiver 6B'. The bore portion 81 and the pipe bore 6c communicate with each other. By providing these slits 82, the proximal end portion side of the coil pipe receiver 6B' is configured to be able to be elastically deformed.

The coil pipe receiver 6B' has the circumferential engaging convex portion 83 in a middle portion of an outer circumference. The engaging convex portion 83 has a semicircular cross-sectional shape and is engagingly inserted and arranged in circumferential grooves having a V-shaped cross-sectional shape that are engaging insertion grooves (described later) formed in the cylinder 7A', that is, V grooves 85a, 85b, and 85c.

An outer diameter dimension of an apex portion of the engaging convex portion 83 of the coil pipe receiver 6B' is ϕ D3. By an external force toward an axis direction acting on the engaging convex portion 83, an outer diameter of the engaging convex portion 83 changes to a small diameter.

Specifically, an outer diameter dimension of the engaging convex portion 83 changes to a diameter smaller than an inner diameter dimension d4 of a receiving bore 7a. By releasing an external force in an axis direction acting on the engaging convex portion 83, the outer diameter dimension of the apex portion of the engaging convex portion 83 of the coil pipe receiver 6B' returns to ϕ D3, that is an original state, by an elastic force. In the present configuration, the engaging convex portion 83 is an urging member.

The cylinder 7A' is integrally fixed to a partition plate 37a by, for example, solder 40. The cylinder 7A' includes the receiving bore 7a that is a moving bore, and the plurality of V grooves 85a, 85b, and 85c that are engaging insertion grooves in which the engaging convex portion 83 is engagingly inserted and arranged are provided in an inner circumferential surface of the receiving bore 7a at predetermined intervals in a longitudinal axis direction.

A wire hole 7c through which the bending operation wire 5 is inserted is formed in a side wall 7b corresponding to a bottom portion of the receiving bore 7a constituting the cylinder 7A'. The wire hole 7c is a through hole for communicating the receiving bore 7a and exterior and is formed on a longitudinal axis of the cylinder 7A'.

In the present configuration, the engaging convex portion 83 of the coil pipe receiver 6B' is engagingly inserted and arranged in the V groove 85c of the cylinder 7A' in an initial state.

When, for example, a compressive force shown by an arrow F5 acts on the coil pipe 4 from the up and down direction bending operation wire 5ud, in this engagingly inserted and arranged state, and the compressive force F5 increases, by an inclined surface of the V groove 85c pressing an outer circumferential surface of the engaging convex portion 83, the engaging convex portion 83 having the outer diameter dimension ϕ D3 gradually has a smaller diameter, and the coil pipe receiver 6B' gradually moves to the proximal end side. Thus, the compressive force acting on the coil pipe 4 from the up and down direction bending operation wire 5ud is released, so that the coil pipe 4 is prevented from being deformed by the compressive force.

When successively, the compressive force acts on the coil pipe 4 from the up and down direction bending operation wire 5ud, and the compressive force shown by the arrow F5 further increases, the coil pipe receiver 6B' further moves to the proximal end side, and the outer diameter dimension of the engaging convex portion 83 pressed by the inclined surface of the V groove 85c becomes substantially the same dimension as the inner diameter dimension d4 of the receiving bore 7a, so that the coil pipe receiver 6B' moves in the receiving bore 7a to the proximal end side. Thus, the compressive force acting on the coil pipe 4 from the up and down direction bending operation wire 5ud is released, so that the coil pipe 4 is prevented from being deformed by the compressive force.

Then, when the compressive force shown by the arrow F5 further increases, the coil pipe receiver 6B' in the receiving bore 7a further moves to the proximal end side, and the engaging convex portion 83 is engagingly inserted in the V groove 85b. Thus, the compressive force acting on the coil pipe 4 from the up and down direction bending operation wire 5ud is released, so that the coil pipe 4 is prevented from being deformed by the compressive force.

In other words, also in the present configuration, by the engaging convex portion 83 of the coil pipe receiver 6B' configured to change the diameter dimension moving in the V grooves 85c, 85b, and 85a formed in the receiving bore 7a in order, the compressive force acting on the coil pipe 4 from the up and down direction bending operation wire 5ud can be released.

In the present configuration, the fixing member receiver is the cylinder 7A', but the fixing member receiver may be a tube having a through hole having the inner diameter dimension d4 of the fixing member receiver. Also, in the present configuration, three V grooves 85 are provided, but the number of the V grooves 85 can be appropriately set and may be three or more.

Further, by providing at a predetermined position on the partition plate 37a a sensor for detecting that the coil pipe receiver 6B' is arranged in the V groove 85a on the proximal end side, a fact that the coil pipe receiver 6B' is moved to a final moving point is grasped, so that the replacement of the coil pipe 4, and the like can be performed at appropriate time.

The present invention is not limited only to the embodiments described above, and various modifications are possible without departing from the scope of the claims.

## Claims

1. A medical appliance comprising a bending mechanism for bending a bending portion of an insertion portion (2a), the medical appliance comprising:
the bending portion (22) provided on a distal end side of a flexible tube (23),
an operation portion (2b) continuously provided at a proximal end of the flexible tube and comprising a bending operation member for operating the bending portion to bend,
a bending operation wire (5) pulled and loosened by bending operation of the bending operation member,
a coil pipe (5), in which the bending operation wire is inserted, having a distal end fixedly provided on the bending portion side and a proximal end provided in the operation portion, and
a coil pipe supporting member comprising an urging member (74) and supporting a proximal end portion of the coil pipe so as to be able to move along a direction in which the bending operation wire extends, wherein the coil pipe supporting member comprises
a fixed supporting shaft (70) integrally fixed to the operation portion and comprising an engaging portion (74) that is the urging member having an outer diameter dimension changeable to a first diameter (d1) dimension and a second diameter (d2) dimension smaller than the first diameter dimension and being elastically deformed from a state of the second diameter dimension to a state of the first diameter dimension, and
a sliding supporting member (60) slidably provided in the operation portion and comprising a sliding portion (61) having a click groove (64) comprising an engaging insertion portion (65) in which the engaging portion (74) is engagingly inserted and a moving portion (66) in which the engaging portion moves, and a coil pipe fixing portion to which the proximal end portion of the coil pipe is directly or indirectly fixed.

2. A medical appliance comprising a bending mechanism for bending a bending portion of an insertion portion (2a), the medical appliance comprising:
the bending portion (22) provided on a distal end side of a flexible tube (23),
an operation portion (2b) continuously provided at a proximal end of the flexible tube and comprising a bending operation member for operating the bending portion to bend,
a bending operation wire (5) pulled and loosened by bending operation of the bending operation member,
a coil pipe (4), in which the bending operation wire is inserted, having a distal end fixedly provided on the bending portion side and a proximal end provided in the operation portion, and
a coil pipe supporting member comprising an urging member (83) and supporting a proximal end portion of the coil pipe so as to be able to move along a direction in which the bending operation wire extends, wherein the coil pipe supporting member comprises
a fixing member (6B') integrally fixed to the proximal end portion of the coil pipe and comprising an engaging portion (83) that is the urging member having an outer diameter dimension changeable to a first diameter (d3) dimension and a second diameter (d4) dimension smaller than the first diameter dimension and being elastically deformed from a state of the second diameter dimension to a state of the first diameter dimension, and
a fixing member receiver (7A') in which a moving bore, in which the fixing member is slidable, and a plurality of engaging insertion grooves (85), in which the engaging portion of the fixing member is engagingly inserted, are provided, the fixing member receiver being fixedly provided in the operation portion.

3. A medical appliance according to claims 1 or 2, wherein the medical appliance is an endoscope overtube.

4. A medical appliance according to claims 1 or 2, wherein the medical appliance is at least one of an endoscope, an endoscope overtube and a treatment instrument inserted through the endoscope.

## Patentansprüche

1. Medizingerät mit einem Biegemechanismus zum Biegen eines Biegeteils eines Einführbereichs (2a), wobei das Medizingerät aufweist:
das Biegeteil (22), das an einer distalen Endseite eines flexiblen Schlauchs (23) vorgesehen ist,
einen Betätigungsbereich (2b), der durchgängig an einem proximalen Ende des flexiblen Schlauchs vorgesehen ist und zum Betätigen des Biegens des Biegeteils ein Biegebetätigungselement aufweist,
einen Biegebetätigungsdraht (5), der durch eine Biegebetätigung des Biegebetätigungselements gezogen und gelockert wird,
ein Spiralrohr (5), in das der Biegebetätigungsdraht eingeführt ist, mit einem auf der Biegeteilseite fest vorgesehenen distalen Ende und einem im Betätigungsteil vorgesehenen proximalen Ende, und
ein Spiralrohr-Haltelement, das ein Presselement (74) aufweist und einen proximalen Endbereich des Spiralrohrs so hält, dass er sich entlang einer Richtung bewegen kann, in welcher sich der Biegebetätigungsdraht erstreckt, wobei das Spiralrohr-Haltelement aufweist:
einen starren Halteschaft (70), der integriert an dem Betätigungsteil befestigt ist und einen Eingriffbereich (74) aufweist, der das Presselement mit einer Außendurchmessergröße darstellt, die sich auf eine erste Durchmessergröße (d1) und eine zweite Durchmessergröße (d2) ändern lässt, die kleiner als die Größe des ersten Durchmessers ist, und aus einem Zustand der zweiten Durchmessergröße in einen Zustand der ersten Durchmessergröße elastisch verformt wird, und
ein Gleithalteelement (60), das verschiebbar im Betätigungsteil vorgesehen ist und ein Gleitteil (61) mit einer Rastnut (64) aufweist, welche einen Eingriff-Einführbereich (65), in den das Eingriffteil (74) eingreifend eingeführt wird, und einen Bewegungsbereich (66), in dem sich das Eingriffteil bewegt, und einen Spiralrohr-Befestigungsbereich aufweist, an dem der proximale Endbereich des Spiralrohrs direkt oder indirekt befestigt ist.

2. Medizingerät mit einem Biegemechanismus zum Biegen eines Biegeteils eines Einführbereichs (2a), wobei das Medizingerät aufweist:
das Biegeteil (22), das an einer distalen Endseite eines flexiblen Schlauchs (23) vorgesehen ist,
einen Betätigungsbereich (2b), der durchgängig an einem proximalen Ende des flexiblen Schlauchs vorgesehen ist und zum Betätigen des Biegens des Biegeteils ein Biegebetätigungselement aufweist,
einen Biegebetätigungsdraht (5), der durch eine Biegebetätigung des Biegebetätigungselements gezogen und gelockert wird,
ein Spiralrohr (4), in das der Biegebetätigungsdraht eingeführt ist, mit einem auf der Biegeteilseite fest vorgesehenen distalen Ende und einem im Betätigungsteil vorgesehenen proximalen Ende, und
ein Spiralrohr-Haltelement, das ein Presselement (83) aufweist und einen proximalen Endbereich des Spiralrohrs so hält, dass er sich entlang einer Richtung bewegen kann, in welcher sich der Biegebetätigungsdraht erstreckt, wobei das Spiralrohr-Haltelement aufweist:
ein Befestigungselement (6B'), das integriert an dem proximalen Endbereich des Spiralrohrs befestigt ist und einen Eingriffbereich (83) aufweist, der das Presselement mit einer Außendurchmessergröße darstellt, die sich auf eine erste Durchmessergröße (d3) und eine zweite Durchmessergröße (d4) ändern lässt, die kleiner als die Größe des ersten Durchmessers ist, und aus einem Zustand der zweiten Durchmessergröße in einen Zustand der ersten Durchmessergröße elastisch verformt wird, und
einen Befestigungselement-Aufnehmer (7A'), in dem eine Bewegungsbohrung, in welcher das Befestigungselement gleiten kann, und eine Vielzahl von Eingriffeinführungsnuten (85), in welche das Eingriffteil des Befestigungselements eingreifend eingeführt ist, vorgesehen sind, wobei der Befestigungselement-Aufnehmer im Betätigungsteil ortsfest vorgesehen ist.

3. Medizingerät nach Anspruch 1 oder 2, wobei das Medizingerät ein Endoskop-Overtube ist.

4. Medizingerät nach Anspruch 1 oder 2, wobei das Medizingerät ein Endoskop, ein Endoskop-Overtube und/oder ein durch das Endoskop eingeführtes Behandlungsinstrument ist.

## Revendications

1. Appareil médical comprenant un mécanisme de cintrage destiné à cintrer une partie de cintrage d'une partie d'insertion (2a), l'appareil médical comprenant :
la partie de cintrage (22) prévue sur un côté d'extrémité distale d'un tube flexible (23),
une partie opérationnelle (2b) prévue de manière continue au niveau d'une extrémité proximale du tube flexible et comprenant un élément d'opération de cintrage destiné à actionner la partie de cintrage pour cintrage,
un câble d'opération de cintrage (5) tiré et relâché par l'opération de cintrage de l'élément d'opération de cintrage,
un tuyau hélicoïdal (5), dans lequel le câble d'opération de cintrage est inséré, ayant une extrémité distale prévue de manière fixe sur le côté de la partie de cintrage et une extrémité proximale prévue dans la partie opérationnelle, et
un élément de support de tuyau hélicoïdal comprenant un élément de sollicitation (74) et supportant une partie d'extrémité proximale du tuyau hélicoïdal de façon à être capable de se déplacer le long d'une direction dans laquelle le câble d'opération de cintrage s'étend, dans lequel l'élément de support de tuyau hélicoïdal comprend
un axe de support fixe (70) fixé solidairement sur la partie opérationnelle et comprenant une partie de mise en prise (74) qui est l'élément de sollicitation et ayant une dimension de diamètre externe chargeable vers une première dimension de diamètre (d1) et une deuxième dimension de diamètre (d2) plus petite que la première dimension de diamètre et étant élastiquement déformé d'un état de la deuxième dimension de diamètre à un état de la première dimension de diamètre, et
un élément de support coulissant (60) prévu de manière coulissante dans la partie opérationnelle et comprenant une rainure d'encliquetage (64) comprenant une partie d'insertion de mise en prise (65) dans laquelle la partie de mise en prise (74) est insérée en engagement et une partie mobile (66) dans laquelle la partie de mise en prise se déplace, et une partie de fixation de tuyau hélicoïdal sur laquelle la partie d'extrémité proximale du tuyau hélicoïdal est directement ou indirectement fixée.

2. Appareil médical comprenant un mécanisme de cintrage destiné à cintrer une partie de cintrage d'une partie d'insertion (2a), l'appareil médical comprenant :
la partie d'insertion (22) prévue sur un côté d'extrémité distale d'un tube flexible (23),
une partie opérationnelle (2b) prévue de manière continue au niveau d'une extrémité proximale du tube flexible et comprenant un élément d'opération de cintrage destiné à actionner la partie de cintrage pour cintrage,
un câble d'opération de cintrage (5) tiré et relâché par l'opération de cintrage de l'élément d'opération de cintrage,
un tuyau hélicoïdal (4), dans lequel le câble d'opération de cintrage est inséré, ayant une extrémité distale prévue de manière fixe sur le côté de la partie de cintrage et une extrémité proximale prévue dans la partie opérationnelle, et
un élément de support de tuyau hélicoïdal comprenant un élément de sollicitation (83) et supportant une partie d'extrémité proximale du tuyau hélicoïdal de façon à être capable de se déplacer le long d'une direction dans laquelle le câble d'opération de cintrage s'étend, dans lequel l'élément de support de tuyau hélicoïdal comprend
un élément de fixation (6B') fixé solidairement sur la partie d'extrémité proximale du tuyau hélicoïdal et comprenant une partie de mise en prise (83) qui est l'élément de sollicitation ayant une dimension de diamètre externe chargeable vers une première dimension de diamètre (d3) et une deuxième dimension de diamètre (d4) plus petite que la première dimension de diamètre et étant élastiquement déformé d'un état de la deuxième dimension de diamètre à un état de la première dimension de diamètre, et
un récepteur d'élément de fixation (7A') dans lequel un alésage mobile, dans lequel l'élément de fixation peut coulisser, et une pluralité de rainures d'insertion de mise en prise (85), dans lesquelles la partie de mise en prise de l'élément de fixation est insérée en engagement, sont prévus, le récepteur d'élément de fixation étant prévu de manière fixe dans la partie opérationnelle.

3. Appareil médical selon la revendication 1 ou 2, dans lequel l'appareil médical est un surtube d'endoscope.

4. Appareil médical selon la revendication 1 ou 2, dans lequel l'appareil médical est au moins l'un parmi un endoscope, un surtube d'endoscope et un instrument de traitement inséré à travers l'endoscope.
